(19) Europäisches Patentamt
European Patent Office
Office européen des brevets

(11) Veröffentlichungsnummer: **0 008 024**
**B1**

(12) # EUROPÄISCHE PATENTSCHRIFT

(45) Veröffentlichungstag der Patentschrift: **25.11.81**

(51) Int. Cl.³: **C 07 C 27/34,** C 07 C 29/76, C 07 C 45/78 // C07C27/20, C07C45/50, C07C67/38

(21) Anmeldenummer: **79102559.6**

(22) Anmeldetag: **20.07.79**

(54) **Verfahren zur Aufarbeitung von Reaktionsgemischen, die bei der Hydroformylierung oder der Carbonylierung anfallen.**

(30) Priorität: **29.07.78 DE 2833469**

(43) Veröffentlichungstag der Anmeldung: **20.02.80 Patentblatt 80/4**

(45) Bekanntmachung des Hinweises auf die Patenterteilung: **25.11.81 Patentblatt 81/47**

(84) Benannte Vertragsstaaten: **BE DE FR GB IT NL**

(56) Entgegenhaltungen: **DE-A-1 041 943** **DE-A-1 493 190**

(73) Patentinhaber: **BASF Aktiengesellschaft, Carl-Bosch-Strasse 38, D-6700 Ludwigshafen (DE)**

(72) Erfinder: **Schwirten, Kurt, Dr.-Chem., Prager Strasse 27, D-6700 Ludwigshafen (DE)**
Erfinder: **Kummer, Rudolf, Dr.-Chem., Kreuzstrasse 6, D-6710 Frankenthal 5 (DE)**
Erfinder: **Richter, Wolfgang, Dr.-Chem., Keplerstrasse 38, D-6700 Ludwigshafen (DE)**

**EP 0 008 024 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Aufarbeitung von Reaktionsgemischen, die bei der Hydroformylierung oder der Carbonylierung anfallen

Die vorliegende Erfindung betrifft ein neues Verfahren zur Aufarbeitung von Reaktionsgemischen, die bei der Hydroformylierung oder der Carbonylierung von olefinisch ungesättigten Verbindungen anfallen und die nichtflüchtige Komplexverbindungen von Metallen der VIII. Gruppe des Periodensystems gelöst enthalten.

Bei der Hydroformylierung oder der Carbonylierung von olefinisch ungesättigten Verbindungen mit Kohlenmonoxid und Wasserstoff bzw. Wasser oder Alkoholen in Gegenwart von Carbonyl-Komplexverbindungen von Metallen der VIII. Gruppe des Periodensystems bei erhöhter Temperatur und unter Druck erhält man, wie allgemein bekannt ist, Reaktionsgemische, die nach verschiedenen Verfahren aufgearbeitet werden. Handelt es sich um die Verwendung flüchtiger Carbonyl-Komplexe, z. B. Dicobaltoctacarbonyl, so ist es erforderlich, diese Carbonylkomplexe zu zerstören, bevor man die Verfahrensprodukte — Aldehyde und Alkohole bei der Hydroformylierung bzw. Säuren und Ester bei der Carbonylierung — vom Rohgemisch abdestilliert, denn andernfalls würden die Verfahrensprodukte durch die Schwermetalle verunreinigt werden.

Verwendet man hingegen solche Komplexverbindungen, in denen ein Teil der Carbonylgruppen durch andere Liganden ersetzt ist, z. B. durch Triphenylphosphin, so bietet dies u. a. den Vorteil, daß man die Verfahrensprodukte unmittelbar vom Rohgemisch abdestillieren kann. Hierbei fällt ein Rückstand aus hochsiedenden organischen Verbindungen an, welcher die unveränderten Katalysatoren gelöst enthält und der deswegen in dieser Form wieder in die Synthesestufe zurückgeführt werden kann, da die hochsiedenden Verbindungen die Hydroformylierungs- bzw. Carbonylierungs-Reaktion nicht stören.

Nachteilig bei dieser Verfahrensweise ist jedoch, daß die Menge des Rückstandes naturgemäß ständig zunimmt, so daß er von Zeit zu Zeit vollständig aufgearbeitet werden muß. Ihn einfach zu verwerfen wäre unwirtschaftlich, besonders wenn er Edelmetallkatalysatoren wie Rhodiumkomplexe enthält, ganz abgesehen davon, daß dies allein aus Umweltgründen nicht möglich wäre.

Nach dem Verfahren der US-PS 3 547 964 behandelt man den Hydroformylierungsrückstand mit wäßrigen Peroxiden, wobei sich aus den Komplexverbindungen der Metalle deren Salze bilden, die sich in der wäßrigen Phase anreichern. Die organische Phase wird verbrannt, während die in der wäßrigen Phase befindlichen Schwermetallsalze mit Kohlenmonoxid und den anderen als Liganden dienenden Verbindungen, z. B. Triphenylphosphin, wieder in die aktive Form der Carbonylkomplexe überführt werden können.

Nach einem anderen Verfahren (DE-OS 2 438 847) wird der Rückstand verbrannt, wonach die Verbrennungsprodukte durch Wasser geleitet werden. Hierbei werden die Schwermetalle zurückgehalten, die man anschließend wieder der Katalysatorregenerierung zuführen kann.

Diese und ähnliche Verfahren, die auf einer chemischen Behandlung des Rückstandes und der Katalysatoren beruhen, sind jedoch apparativ aufwendig, bieten zahlreiche verfahrenstechnische Probleme und sind wirtschaftlich allein deswegen unbefriedigend, weil der Neubildung des Katalysators dessen Zerstörung vorausgehen muß. Häufig stellt jedoch nicht allein die Aufbereitung des hochsiedenden Rückstandes ein technisches Problem dar, sondern bereits die Abtrennung der Verfahrensprodukte. Sind diese — auch unter vermindertem Druck — nämlich wie höhere Aldehyde, Alkohole, Säuren oder Ester selber hochsiedend, so können sich die Katalysatoren, auch wenn sie stabilisierende Liganden wie tertiäre Phosphine enthalten, bei den Destillationstemperaturen zu den Metallen zersetzen. Soweit die Metalle überhaupt zurückgewonnen werden können und sich nicht an den Wandungen der Destillationsapparaturen niederschlagen, müssen sie auf umständliche Weise erst wieder in die aktiven Katalysatoren überführt werden.

Zur Trennung von Stoffgemischen war es aus der DE-AS 1 493 190 in allgemeiner Form bekannt, Gase in überkritischem Zustand für die Extraktion zu verwenden. Nach Beispiel 3 dieser DE-AS verbleiben salzartige Verbindungen wie Ammoniumchlorid bei der Extraktion von Ammoniumchlorid/Paraffinöl-Mischungen mit Äthylen hierbei im Rückstand, werden also von dem überkritischen Gas nicht in die Extraktphase aufgenommen. Andere Metallverbindungen wie Al-sec.-butylat (Beispiel 2) und der Mg-Komplex Chlorophyll (Beispiel 15) gelangen hingegen in die Extraktphase. Das Verfahren der DE-AS 1 493 190 läßt demnach nicht erwarten, daß man nicht-salzartige Metallverbindungen, insbesondere nicht-flüchtige Metallkomplexverbindungen, hiernach von schwerflüchtigen organischen Bestandteilen abtrennen kann.

Der Erfindung lag die Aufgabe zugrunde, Reaktionsgemische, die bei der Hydroformylierung oder der Carbonylierung von olefinisch ungesättigten Verbindungen mittels nicht-flüchtiger gelöster Komplexverbindungen von Metallen der VIII. Gruppe des Periodensystems anfallen, in die Komplexverbindungen oder konzentrierte Lösungen dieser Komplexverbindungen einerseits und die übrigen Komponenten andererseits zu trennen.

Demgemäß wurde ein Verfahren zur Aufarbeitung von Reaktionsgemischen, die bei der Hydroformylierung oder der Carbonylierung von olefinisch ungesättigten Verbindungen anfallen und die sich aus

1. Bestandteilen, die niedriger als die hauptsächlichen Hydroformylierungs- bzw. Carbonylierungsprodukte sieden (»Leichtsieder«),
2. den hauptsächlichen Hydroformylierungs- bzw. Carbonylierungsprodukten (»Verfahrensprodukte«),
3. Bestandteilen, die höher als Leichtsieder und die Verfahrensprodukte sieden (»Hochsieder«) und
4. nichtflüchtigen gelösten Komplexverbindungen von Metallen der VIII. Gruppe des Periodensystems

zusammensetzen, gefunden, welches darin besteht, daß man

a) die Leichtsieder allein oder zusammen mit den Verfahrensprodukten oder einem Teil der Verfahrensprodukte destillativ vom Reaktionsgemisch abtrennt und
b) von den verbleibenden Lösungen die Verfahrensprodukte oder die Verfahrensprodukte einschließlich eines Teiles der Hochsieder oder, falls die Verfahrensprodukte bereits destillativ abgetrennt wurden, einen Teil der Hochsieder mit Kohlendioxid, einem $C_2-C_4$-Paraffin, einem $C_2-C_4$-Olefin oder einem unter Normalbedingungen gasförmigen Halogenwasserstoff oberhalb der kritischen Temperatur und oberhalb des kritischen Druckes dieser Verbindungen extrahiert.

Kritische Temperaturen und der dazugehörige kritische Druck eines Stoffes sind bekanntlich diejenigen Grenzwerte, oberhalb derer eine Umwandlung vom gasförmigen in den flüssigen Aggregatzustand nicht mehr möglich ist. Hier verhalten sich die Gase in mancher Hinsicht wie Flüssigkeiten, und die Erfindung beruht auf der überraschenden Entdeckung, daß sich die organischen Anteile der aus der Hydroformylierung oder der Carbonylierung stammenden Reaktionsgemische einschließlich der hochsiedenden Rückstände weitgehend in der Quasi-Flüssigkeit des überkritischen Gases lösen und somit, wie mit einer echten Flüssigkeit, extrahiert werden können. Die Katalysatoren hingegen werden praktisch nicht von den überkritischen Gasen aufgenommen und bleiben bei der Extraktion fast vollständig zurück. Der verbleibende katalysatorhaltige Rückstand kann dann unmittelbar in die Synthesestufe zurückgeführt werden. Im allgemeinen richtet man es hierbei so ein, daß man 20 bis 80 Gewichtsprozent des hochsiedenden Rückstandes auf die erfindungsgemäße Weise extraktiv entfernt, und zwar entweder zusammen mit den Verfahrensprodukten oder, wenn diese ohne Schädigung des Katalysators destillierbar sind, nach deren vorheriger destillativer Abtrennung.

In der folgenden Tabelle sind die abgerundeten kritischen Daten einiger der definitionsgemäßen Verbindungen aufgeführt.

| | Kritische Temp. °C | Kritischer Druck bar |
|---|---|---|
| $CO_2$ | 31 | 74 |
| Äthan | 32 | 49 |
| Äthylen | 10 | 51 |
| Propan | 97 | 47 |
| Butan | 152 | 38 |
| Isobuten | 145 | 40 |
| $CClF_3$ | 30 | 39 |

Bevorzugt werden diejenigen Extraktionsmittel, deren kritische Temperatur zwischen (−50) und 200°C, besonders zwischen 0 und 150°C liegt. Die entsprechenden Arbeitstemperaturen liegen zweckmäßigerweise jeweils etwa 5 bis 50°C höher. Für den Druck wählt man bevorzugt Werte zwischen 40 und 200 bar oberhalb des kritischen Druckes. Für die Aufnahme von 1 kg organischer Substanz (Verfahrensprodukte und/oder Rückstand) benötigt man in der Regel rund 2 bis 20 kg des definitionsgemäßen Extraktionsmittels.

Nach den bisherigen Beobachtungen werden sämtliche definitionsgemäßen Metallverbindungen von den Gasen im überkritischen Zustand nicht aufgenommen, so daß es auf die chemische Natur dieser Verbindungen hinsichtlich ihres Verhaltens bei der Extraktion nicht ankommt. Es seien daher

lediglich diejenigen Metallkomplexverbindungen genannt, die im Rahmen der Oxo-Synthese bisher die größte Bedeutung erlangt haben, nämlich die Komplexe des Cobalts und vor allem des Rhodiums wie $Co_2(CO)_4(PR_3)_2$, $Rh_2(CO)_6(PR_3)_2$, $HCo(CO)_2(PR_3)_2$, $HCo(CO)_3(PR_3)$, $Co_2(CO)_6(PR_3)_2$ und Pyridinium-$[Co(CO)_4]^-$, wobei R gleiche oder verschiedene Kohlenwasserstoffreste mit 4 bis 16 C-Atomen bedeutet. Gebräuchliche Liganden dieser Art sind solche, in denen die Reste R im Phosphin n-Butyl, n-Hexyl, n-Octyl, n-Decyl und Phenyl bedeuten.

Diese Verbindungen liegen, berechnet auf ihren Metallgehalt, in den hochsiedenden Rückständen je nach ihrem Anreicherungsgrad im allgemeinen in Konzentrationen von 0,01 bis 3 Gewichtsprozent vor.

Bei der Hydroformylierung olefinisch ungesättigter Verbindungen erhält man, wie allgemein bekannt ist, je nach den Verfahrensbedingungen neben leicht flüchtigen Nebenprodukten (z. B. paraffinischen Verbindungen aus der Hydrierung der olefinischen Verbindungen) Aldehyde und Alkohole in unterschiedlichen Mengen:

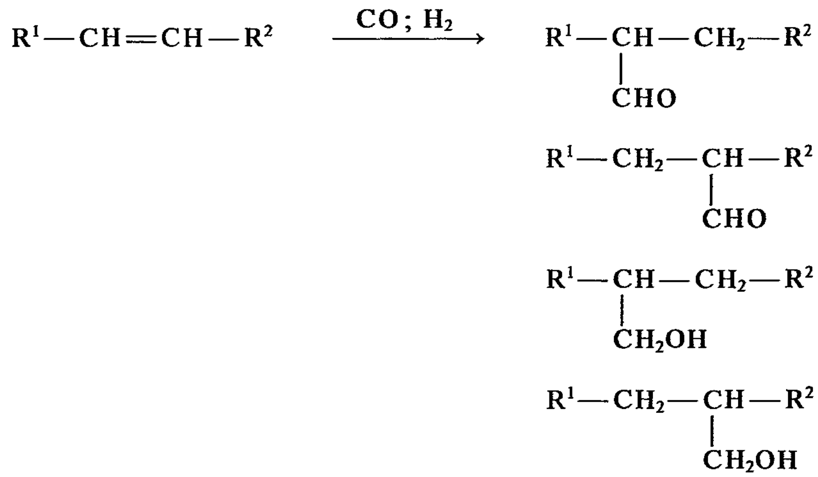


$R^1$ und $R^2$: Wasserstoff oder organische Reste

Aus diesen primären Verfahrensprodukten bilden sich als Nebenprodukte Aldolisierungs- und Acetalisierungsprodukte sowie ferner — durch Disproportionierung der Aldehyde zu Säuren und Alkoholen — Ester dieser Säuren. Diese Verbindungen sind schwer flüchtig und bilden im wesentlichen den hier in Rede stehenden Rückstand aus der Oxo-Synthese.

Bei der Carbonylierung im engeren Sinne (häufig wird auch die Hydroformylierung zu den Carbonylierungs-Reaktionen allgemein gezählt) handelt es sich um eine analoge Reaktion, bei der man Säuren oder Ester erhält, je nachdem, ob man neben dem Kohlenmonoxyd Wasser oder Alkohole als weiteren Reaktionspartner verwendet:

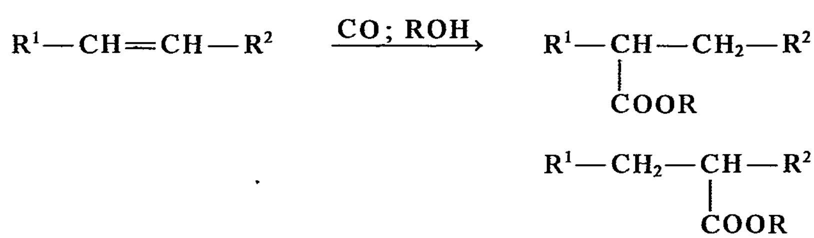


$R^1$, $R^2$ und R: Wasserstoff oder organische Reste

Auch hier fallen diverse hochsiedende Nebenprodukte an, die ihrer chemischen Natur nach denjenigen aus der Hydroformylierung ähneln, da die Reaktion in reduzierender Atmosphäre (CO) stattfindet.

Besondere Bedeutung hat das erfindungsgemäße Verfahren im Zusammenhang mit denjenigen Synthesen unter Verwendung von Cobalt- oder Rhodiumkatalysatoren, die im großtechnischen Maßstab vorgenommen werden, also bei der Hydroformylierung und der Carbonylierung von Äthylen, Propylen und $C_8-C_{14}$-Olefinen. Auf eine Tonne der erwähnten Verfahrensprodukte fallen hierbei etwa 10 bis 50 kg hochsiedender Rückstand an.

Im Falle der Rückstandsaufbereitung nimmt man die Extraktion in einem senkrecht stehenden Autoklaven vor, den man zweckmäßigerweise zu 10 bis 30% seines Volumens mit dem Rückstand beschickt. Das restliche, oberhalb des Rückstandes befindliche Volumen ist dann unter den

überkritischen Bedingungen mit dem Gas ausgefüllt. Zur Extraktion leitet man zusätzliches Gas — immer unter den überkritischen Bedingungen — von unten durch den Rückstand und zieht es nach Maßgabe seiner Zufuhr oben, wo es sich mit dem Rückstand angereichert hat, ab. Über die optimale Extraktionsgeschwindigkeit lassen sich keine absoluten Werte angeben, da sie von der Art des Rückstandes und des Gases sowie von Temperatur, Druck und den apparativen Gegebenheiten abhängig sind. Man kann sie aber unschwer ermitteln, indem man zunächst mit einem geringen Gasdurchsatz beginnt, diesen Durchsatz pro Zeiteinheit stufenweise erhäht und die hierbei jeweils extrahierten Rückstandsmengen bestimmt. Der optimale Durchsatz ist dann derjenige, bei welchem die Rückstandsmenge nach abermaliger Erhöhung des Durchsatzes nicht mehr nennenswert zunimmt.

Wie bei normalen Extraktionen wird der Wirkungsgrad durch feine Verteilung des Extraktionsmittels, also des Gases, und durch Füllkörper zur Beschleunigung der Gleichgewichtseinstellung erhöht.

Der Austrag erfolgt über ein Entspannungsventil in einen Abscheider, wobei es genügt, das Gas, da es zweckmäßigerweise im Kreis geführt wird und zu diesem Zweck erneut komprimiert werden muß, auf einen Druck unterhalb des kritischen Druckes zu entspannen. Hierbei verliert das Gas den extrahierten Hochsieder, der dann aus dem Abscheider abgezogen werden kann. Enthält der Hochsieder noch geringe Mengen an Metall, so liegt dies in aller Regel daran, daß die Metallverbindung nicht extrahiert, sondern mechanisch mitgerissen wurde. Durch Verlangsamung des Extraktionsvorganges läßt sich dieser Metallgehalt aber praktisch gänzlich vermeiden. Die Raffinatphase kann nach der Extraktion unmittelbar in die Synthesestufe zurückgeführt werden. Sonstige, im Hochsieder enthaltene Verbindungen, nämlich überschüssige Komplexbildner wie Triphenylphosphin gelangen teils in die Extrakt-, teils in die Raffinatphase.

Analog arbeitet man, wenn — nachdestillativer Abtrennung der Leichtsieder — nicht nur der Rückstand, sondern das gesamte Reaktionsgemisch auf die erfindungsgemäße Weise aufgearbeitet werden soll. Da als Regel gilt, daß für Verbindungen mit relativ niedrigem Molekulargewicht ein relativ geringer Druck des überkritischen Gases für die Extraktion ausreicht und daß mit steigendem Molekulargewicht steigende Drücke erforderlich sind, lassen sich durch Wahl eines geeigneten Druckes zunächst die Verfahrensprodukte und danach, unter höherem Druck, der Rückstand extrahieren. Zur Reinigung der Verfahrensprodukte, soweit diese überhaupt noch erforderlich ist, kann das Extraktionsverfahren wiederholt werden, oder man trennt die Verfahrensprodukte wie üblich destillativ von den Hochsiedern ab.

Überraschenderweise werden die Katalysatoren durch die erfindungsgemäße Behandlung ihrer Lösungen reaktiviert, wobei sie die gleiche Aktivität zeigen, wie frisch hergestellter Katalysator. Im Hinblick darauf, daß die Aktivität der Katalysatoren nach mehreren Zyklen der unbehandelten Hochsieder bis auf etwa 40% ihres Anfangswertes zurückfallen kann, liegt hierin ein besonderer Vorteil des Verfahrens. Ein unmittelbares Maß für die Aktivität des Katalysators ist hierbei der Umsatz des Olefins zu den Hydroformylierungsprodukten pro Zeiteinheit.

## Beispiel 1

250 g eines hochsiedenden Rückstandes, welcher nach Abtrennung der Verfahrensprodukte (Butyraldehyde und Buanole) und der leicht flüchtigen Bestandteile bei der Hydroformylierung von Propylen mittels eines Rhodiumkatalysators anfiel und der 209 ppm Rh (=52,3 mg Rh) in Form des Komplexes $HRh(CO)(PPh_3)_3$ sowie 7 Gewichtsprozent freies Triphenylphosphin $PPh_3$ enthielt, wurden in einen Autoklaven von 1,5 l Inhalt bei 25°C und 100 bar in der Weise mit Äthylen im überkritischen Zustand extrahiert, daß man im Laufe von 10 Stunden unter Konstanthaltung von Druck und Temperatur 37 kg (= 2,2 Nm³) Äthylen kontinuierlich von unten durch den Rückstand zuführte und die entsprechende Menge durch ein Entspannungsventil abzog.

Bei der Entspannung des Äthylens kondensierten 169 g (= 68 Gew.-%) des extrahierten Rückstandes, der 2,1 mg Rh enthielt. Die restliche Menge des Rückstandes verblieb, zusammen mit 50,2 mg Rh im Autoklaven. 96% des Rhodiums, und zwar in Form des aktiven Komplexes, konnten somit zurückgewonnen werden. Dieser Teil des Rückstandes eignete sich dazu, ohne weitere Behandlung in die Hydroformylierungsstufe zurückgeführt zu werden.

Die Wirkung dieses behandelten Rückstandes wurde mit der Wirkung der gleichen Menge unbehandelten Rückstandes gleicher Rh-Konzentration bei der Hydroformylierung verglichen. Hierbei zeigte sich, daß der Katalysator des unbehandelten Rückstandes zu Beginn lediglich eine Aktivität von etwa 70% des Katalysators im behandelten Rückstand aufwies. Als Maß für die Aktivität diente der Umsatz des Propylens zu den Butylraldehyden und -alkoholen unter somit gleichen Hydroformylierungsbedingungen.

## Beispiel 2

Ein Hochdruckrohr von 1,2 l Inhalt wurde von unten pro Stunde kontinuierlich mit 135 g eines hochsiedenden Rückstandes aus der in Beispiel 1 genannten Synthese und 940 g Kohlendioxid

(= 470 Nl) beschickt, wobei das Gas bei 45°C unter einem Druck von 130 bar stand. Aus dem oben entnommenen Gas fielen, nach Entspannung, pro Stunde 18 g praktisch katalysatorfreier Rückstand an. Insgesamt wurden auf diese Weise 2050 g Hochsieder, die 512 mg Rh enthielten, behandelt. Davon blieben 595 g zurück, die rund 94% des Rhodiums enthielten.

## Beispiel 3

80 g eines hochsiedenden Rückstandes, welcher nach Abtrennung der Verfahrensprodukte (Pentadecanale und -decanole) und der leicht flüchtigen Bestandteile bei der Hydroformylierung von Tetradec-1-en mittels eines Cobaltcarbonyl/Trioctylphosphin-Katalysators anfiel und der 3,2 g Cobalt in Form des Komplexes enthielt, wurden analog Beispiel 1 bei 20°C und 110 bar mit Äthylen extrahiert. Hierbei ließen sich 62% der Hochsieder entfernen, und in dem restlichen Rückstand verblieben 96% des Cobalts.

Der Rückstand bestand hauptsächlich aus Diolen und Acetalen mit diesen Diolen. Die Diole ihrerseits resultierten aus der Bis-Hydroformylierung von Diolefinen, welche das Tetradec-1-en üblicherweise in geringen Mengen begleiten.

## Beispiel 4

375 g eines Reaktionsgemisches, welches aus der Hydroformylierung von Octadec-1-en mittels des Komplexes $HRh(CO)(PPh_3)_3$ entstammte und welches 6 Gew.-% Octadec-1-en, 87 Gew.-% Nonadecanole, 7 Gew.-% freies Triphenylphosphin sowie 65 mg Rhodium enthielt, wurden analog Beispiel 1 bei 100 bar und 27°C mit Äthylen extrahiert. Hierbei gingen Octadecen und die Nonadecanole quantitativ in die Extraktphase, die lediglich 0,6 mg Rhodium enthielt.

Der katalysatorhaltige Rückstand wurde in die Hydroformylierungsstufe zurückgeführt. Die Aktivität des Katalysators entsprach derjenigen eines frisch zugesetzten Rh-Komplexes.

## Beispiel 5

Analog Beispiel 4 wurden 330 g eines Reaktionsgemisches, welches aus der Hydroformylierung von Tetradec-1-en mit $CO_2(CO)_4(P\text{-octyl}_3)_4$ stammte, bei 110 bar und 25°C mit Äthylen extrahiert. Nach Entspannung fielen 290 g Extraktphase an, die größtenteils aus Pentadecanolen und -decanalen bestand und außerdem noch geringe Mengen an Tetradecan, nicht umgesetztem Tetradecen, und 2 Gew.-% des Cobalts enthielt.

Der Rückstand, welcher 98 Gew.-% des Cobalts enthielt, wurde in die Hydroformylierungsstufe zurückgeführt, wo der Katalysator sich voll aktiv erwies.

## Beispiel 6

In der in Beispiel 2 beschriebenen Vorrichtung für die kontinuierliche Extraktion wurden stündlich 120 g des Hydroformylierungsgemisches aus Beispiel 4 bei 43°C und 110 bar mit 600 g (rd. 300 Nl) Kohlendioxid extrahiert. Hierbei fielen stündlich zwischen 96 und 102 g Extraktionsphase der gleichen Zusammensetzung wie in Beispiel 4 an, deren Rhodiumgehalt lediglich 0,4 mg betrug.

Der Rückstand wurde in die Hydroformylierungsstufe, worin sich der Katalysator voll aktiv erwies, zurückgeführt.

## Beispiel 7

5000 g eines Rückstandes, welcher nach destillativer Abtrennung des Methylpropionates bei der Carbonylierung von Äthylen mit Kohlenmonoxid und Methanol mittels $\text{Pyridinium}^{\oplus} — Co(CO)_4)^{\ominus}$ anfiel und der 100 g Co enthielt, wurde bei 40°C und 110 bar mit 25 kg (rd. 12,5 $Nm^3$) $CO_2$ extrahiert. Die Menge des hierbei anfallenden Rückstandes betrug 720 g. 93 g Cobalt verblieben in diesem Rückstand, 7 g gelangten in die Extraktphase.

Der Rückstand wurde in die Carbonylierungsstufe zurückgeführt, wo sich der Katalysator als voll aktiv erwies.

**Patentanspruch**

Verfahren zur Aufarbeitung von Reaktionsgemischen, die bei der Hydroformylierung oder der Carbonylierung von olefinisch ungesättigten Verbindungen anfallen und die sich aus

1. Bestandteilen, die niedriger als die hauptsächlichen Hydroformylierungs- bzw. Carbonylierungsprodukte sieden (»Leichtsieder«),
2. den hauptsächlichen Hydroformylierungs- bzw. Carbonylierungsprodukten (»Verfahrensprodukte«),
3. Bestandteilen, die höher als Leichtsieder und die Verfahrensprodukte sieden (»Hochsieder«) und
4. nichtflüchtigen gelösten Komplexverbindungen von Metallen der VIII. Gruppe des Periodensystems

zusammensetzen, welches darin besteht, daß man

a) die Leichtsieder allein oder zusammen mit den Verfahrensprodukten oder einem Teil der Verfahrensprodukte destillativ vom Reaktionsgemisch abtrennt und
b) von den verbleibenden Lösungen die Verfahrensprodukte oder die Verfahrensprodukte einschließlich eines Teiles der Hochsieder oder, falls die Verfahrensprodukte bereits destillativ abgetrennt wurden, einen Teil der Hochsieder mit Kohlendioxid, einem $C_2-C_4$-Paraffin, einem $C_2-C_4$-Olefin oder einem unter Normalbedingungen gasförmigen Halogenwasserstoff oberhalb der kritischen Temperatur und oberhalb des kritischen Druckes dieser Verbindungen extrahiert.

**Claim**

A process for working up reaction mixtures which are obtained in the hydroformylation or carbonylation of olefinically unsaturated compounds and which are composed of

1. constituents which boil at a lower temperature than the main hydroformylation or carbonylation products (»low-boiling constituents«),
2. the main hydroformylation or carbonylation products (»products of the process«),
3. constituents which boil at a higher temperature than the low-boiling constituents and the products of the process (»high-boiling constituents«), and
4. non-volatile dissolved complex compounds of metals of Group VIII of the periodic table,

which comprises

(a) separating off the low-boiling constituents alone or together with the products of the process or with part of the products of the process from the reaction mixture by distillation, and
(b) extracting the products of the process or the products of the process including part of the high-boiling constituents or, if the products of the process have already been separated off by distillation, part of the high-boiling constituents form the solutions which are left, with carbon dioxide, a $C_2-C_4$-paraffin, a $C_2-C_4$-olefin or a hydrogen halide which is normally gaseous at above the critical temperature and above the critical pressure of these compounds.

**Revendication**

Procédé pour traiter des mélanges de réaction obtenus à l'hydroformylation ou à la carbonylation de composés à insaturation oléfinique et qui sont composés de:

1. des constituants bouillant plus bas que les produits principaux d'hydroformylation ou de carbonylation (»produits légers«),
2. les produits principaux d'hydroformylation ou de carbonylation (»produits recherchés«),
3. des constituants bouillant plus haut que les produits légers et que les produits recherchés (»produits lourds«) et
4. des composés complexes non volatils de métaux du groupe VIII de la Classification Périodique, à l'état dissous,

procédé qui consiste en ce que:

a) on sépare du mélange de réaction, par distillation, les produits légers seuls ou avec les produits recherchés ou une partie des produits recherchés et
b) à partir des solutions résiduelles, on extrait les produits recherchés ou les produits recherchés

avec une partie des produits lourds ou bien, lorsque les produits recherchés ont déjà été séparés par distillation, une partie des produits lourds par l'anhydride carbonique, une paraffine en C2—C4, une oléfine en C2—C4 ou un halogénure d'hydrogène à l'état gazeux dans les conditions normales, au-dessus de la température critique et au-dessus de la pression critique de ces composés.